# EUROPEAN PATENT SPECIFICATION

(11) **EP 0 809 472 B1**
(45) Date of publication and mention of the grant of the patent: **24.04.2002**
(21) Application number: 96946122.7
(22) Date of filing: 18.10.1996
(51) Int. Cl.: A61B 17/68

(54) **INTRAMEDULLARY CAVITY NAIL FOR FEMUR ELONGATION**
MARKRAUMNAGEL FÜR DEN EINSATZ ZUR FEMURVERLÄNGERUNG
CLOU DE CAVITE INTRAMEDULLAIRE DESTINE A L'ELONGATION DU FEMUR

(30) Priority: 21.10.1995 DE 29516653 U; 01.12.1995 DE 19544820
(43) Date of publication of application: 03.12.1997
(73) Proprietor: Pennig, Dietmar, Dr., 50935 Köln (DE)
(72) Inventor: Pennig, Dietmar, Dr., 50935 Köln (DE)
(74) Representative: Botti, Mario
(86) International application number: EP9604539
(87) International publication number: WO9715176

(56) References cited:
- EP-A- 0 639 352
- DE-U- 9 115 200
- FR-A- 2 289 155
- US-A- 2 614 559

## Description

### Technical Field

The invention relates to an intramedullary cavity nail intended for use in elongation of the femur.

### Background Art

According to known methods and apparatus, femur elongations are carried out with the aid of an external securing device which is externally secured to the femur, the femur being separated in the central area between the fixing pins of the securing device. By means of staged adjustment of the spacing between the two fixing points of the securing device, elongation of the femur is effected. After the final or desired length has been reached, the bone must be stabilised in its final length until a sufficiently secure connection has been achieved, and the bone in its entirety has achieved the necessary load-bearing capacity.

DE 9115200 U discloses a locking nail for a plurality of bone cavities according to the first part of claim 1. It is hollow and straight and has an outer diameter which is for the most part smaller than the inner diameter of the corticalis. In this way, enlarging the bone cavity is generally not necessary. The nail is also provided in a plurality of lengths and is suited for dynamic medical care as well as for subjecting compression and/or distraction forces.

US 2,614,559 discloses an intramedullary bar of suitable metal provided with a plurality of spaced holes and companionate screws having a bore diameter with an outer diameter adapted to pass through the holes. The bar may be placed in the medullary cavity of a bone and held therein in spaced relationship to the bone by means of screws passing through the bone and through any substantial fragments thereof, so that the broken bone and/or fragments thereof, may be held in their true position while the healing processes are proceeding to completion.

EP-A-0639352 discloses a bone treating device comprising a nail which can be inserted in the bone and whose central axis is crossed by perforations for the reception of screws, bolts and similar pin-like elements. The nail is provided on one end with at least one round hole and, at the other end, with a longitudinal slot for reception of at least one screw, bolt or similar pin-like element which is slideable therein.

### Disclosure of the Invention

The invention according to one preferred aspect therefore proposes to achieve stabilisation of the bone by means of an intramedullary cavity nail for insertion into the bone medullar cavity which also for example affords the possibility that, after the final position has been reached, the awkward external securing device can be removed, the bone despite this being sufficiently supported and secured by the intramedullary nail.

The use of conventional intramedullary nails for this purpose is not possible, as on the one hand the medullar cavity nails are far too long and on the other hand the conventional medullar cavity nails are adapted in their configuration to the curvature of the femur. External securing devices are in principle designed as straight rails, and are therefore difficult to use in conjunction with curved intramedullary nails.

An object underlying the invention according to one preferred aspect is therefore to propose an intramedullary nail which is specially adapted to its new purpose.

According to a preferred aspect of the invention, there is provided an intramedullary nail which is essentially straight in shape, i.e. does not have the curvature adapted to the femur, and is only of a length which is shorter than the distance from the trochanter minor to the knee joint. For example, the nail has a length corresponding to 50% of the length of the femur. In this case there are also provided, in the proximal and distal areas of the nail, locking apertures which pass entirely through the nail, and through which appropriate screws or pins can be inserted. In order to insert the nail deep into the bone, a connector device is provided at the end of the nail, to which a handling means can be connected which connects non-rotatably with the nail, so that insertion of the nail is possible with the aid of rotary movements via the handling means. When the nail reaches its desired position within the femur, the handling means can be removed.

The proximal end of the nail then, contrary to known devices, does not abut on the trochanter major, but penetrates so as to be positioned relatively deep into the medullar cavity, seen in the longitudinal axis of the femur.

The upper locking means, i.e. the locking apertures of the proximal end of the nail, extend in the frontal plane, so that insertion of locking nails or pins into the appropriate locking apertures can be effected from the side. In comparison, the locking apertures in the nail in the distal terminal area of the nail extend in the sagittal plane, so that insertion of the locking nails therein can be effected from the front. This locking from the front to the back can in the present case be carried out in a technically simpler manner, as in this case the external securing device does not lie in the region where there is normally positioned an X-ray image converter.

According to a further feature of the invention, the intramedullary nail tapers slightly conically, e.g. 6 cm from the proximal end or e.g. roughly in the centre of its length, and thus adapts better to the natural formation of a femur.

The nail is preferably tubular in form, and in addition tapers either conically or in a curved configuration at its distal end, simplifying its introduction into the femur and thus into the medullar cavity.

### Brief Description of Drawings

Preferred embodiments of the invention, given by way of example, are described in the following detailed description with reference to the drawings, in which:

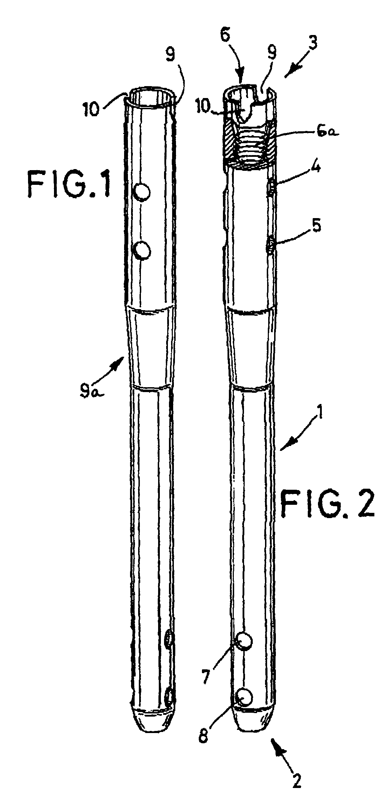

Fig. 1 is a perspective view of an intramedullary nail in accordance with one preferred aspect of the invention;
Fig. 2 is a perspective view of the intramedullary nail according to Fig. 1, in which the upper end is shown in a cutaway section view, and the nail has been rotated through approximately 90°; and
Fig. 3 is a sectional elevation view of the nail of Figs. 1 and 2 inserted into the medullar cavity of a femur during elongation thereof.

### Best Modes for Carrying out the Invention

In the drawing Figures, the reference numeral 1 indicates an intramedullary nail which has a distal end 2 and a proximal end 3. In the region of the proximal end 3 there is provided a connector device 6, which in the embodiment shown is represented by an internal tapping with a hollow upper portion and a reduced diameter lower threaded portion 6a. There are also provided in the upper region slots 9 and 10, a plurality of which can be provided, and which serve to reliably secure a handling means in a non-rotatable manner, said handling means can be connected to the connector device 6, and enables the intramedullary nail to be inserted deep into the femur. The length of the intramedullary nail 1 in this respect can correspond to only 50% of the overall length of the femur, but in any case is shorter than the length of the femur between the trochanter minor and the knee joint. There are provided in the proximal area 3 locking apertures 4 and 5 which extend in the frontal plane when the nail is inserted into the femur, while the locking apertures 7 and 8 provided at the distal end 2 extend in the inserted state in the sagittal plane, so that the locking screws, nails, or pins can be inserted into the apertures 4 and 5 from the side, while the locking means for apertures 7 and 8 can be inserted from the front, seen from the front of the bone.

As the medullar cavity expands from the distal to the proximal end, the nail 1 is also adapted to this, by having a conical taper 9a which is provided roughly 6 cm from the proximal end, in the embodiment shown, roughly in the middle. The nail 1 is preferably in the form of a tubular member.

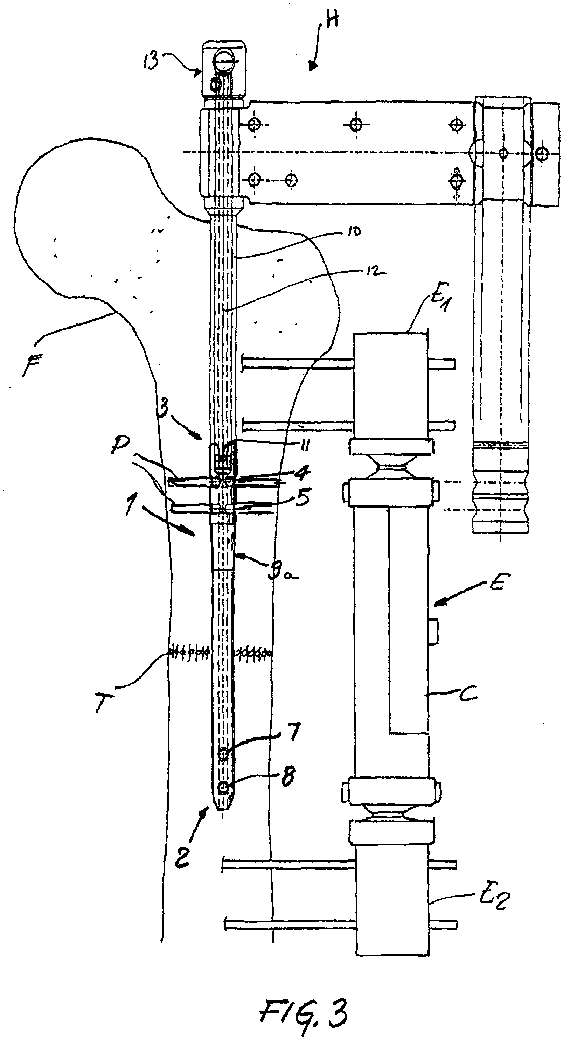

Fig. 3 shows the intramedullary nail 1 used in combination with an external fixator E of a conventional type with an elongatable central body C and a pair of clamping means E1,E2. The external fixator may be for example a fixator of the series 10,000 of Orthofix s.r.1., Italy.

Fixator E is secured to femur bone F by means of two pairs of pins inserted at opposite end portions of the bone. Nail 1 is inserted into the medullar cavity of the femur F with the help of handling means releasably connected to the connector device of the nail. The handling means are in the form of a handling device H including a hollow support rod 10 having oppositely arranged protrusions 11 arranged at an end thereof for being lodged in the slots 9,10, so that the support rod is non-rotatably connected to the nail. The handling device H further includes a tie-rod 12 rotatably accommodated inside the hollow support rod 10, and a knob connected to the tie-rod 12 for rotation thereof. The end of the tie rod is threaded for being releasably screwed into the threaded portion 6a of the connector device 6. Screwing of the threaded end of the tie rod 12 into the threaded portion 6a releasably connects the lower end of the support rod 10 into the upper hollow portion of the connector device 6, with the protrusions 11 arranged in the slots 9,10. Unscrewing of the tie rod 12 from the threaded portion 6a permits the handing device H to be released from the nail 1 by being pulled away therefrom. Once the nail 1 is inserted in the intramedullary cavity of the femur F, it is secured to the bone by means of one or two pins P passing through proximal holes 4,5 and leaving the distal end 2 of the nail free. The bone F is ostheotomized at T, and a tensioning device, not shown in the figure, but of a conventional type and in a conventional manner is mounted to the fixator F in order to apply incremental stretching forces to the bone for elongation thereof along its longitudinal axis up to the desired final length.

Once the final length has been reached, the distal end 2 of the nail is secured to the bone by means of a second pair of pins passing through the distal holes 7,8 while the fixator F can be removed from the bone, thereby advantageously leaving only the nail 1 to stabilise the bone during healing thereof.

## Claims

1. A intramedullary nail (1) for elongation of the femur suitable to be inserted into the bone medullar cavity between the tronchanter minor and the knee joint of the femur comprising an elongate rectilinear body having at a proximal end (3) and at a distal end (2) thereof respective through apertures (4,5;7,8) for receiving pins (P) or screws for fixing the ends (2,3) to the femur **characterized in that** the proximal through apertures (4,5) have longitudinal axes which extend in a first plane and the distal through apertures (7,8) have longitudinal axes which extend in a second plane, said first and second planes being substantially mutually perpendicular.

2. A nail (1) according to claim 1 further comprising a conical taper (9a) positioned centrally at the elongate body between the proximal and distal ends (2,3).

3. A nail according to claim 2, in which the taper (9a) is conical outwardly and is positioned 6 centimetres from the proximal end.

4. A nail according to claim 1, in which said elongate body has a tubular shape.

## Patentansprüche

1. Intramedullärer Nagel (1) zur Verlängerung des Oberschenkels, der geeignet ist, um in den medullären Knochenhohlraum zwischen den Trochanter minor und das Kniegelenk des Oberschenkels insertiert zu werden, umfassend einen verlängerten geradlinigen Körper mit an einem proximalen Ende (3) und an einem distalen Ende (2) davon jeweils Durchöffnungen (4, 5; 7, 8) zur Aufnahme von Nägeln (P) oder Schrauben zur Fixierung der Enden (2,3) an dem Oberschenkel, **dadurch gekennzeichnet, daß** die proximalen Durchöffnungen (4,5) Längsachsen haben, die sich in einer ersten Ebene erstrekken und die distalen Durchöffnungen (7,8) Längsachsen haben, die sich in einer zweiten Ebene erstrecken, wobei die erste und zweite Ebene im wesentlichen senkrecht zueinander sind.

2. Nagel nach Anspruch 1, weiter umfassend einen konischen Kegel (9a), der zentral an dem verlängerten Körper zwischen dem proximalen und distalen Ende (2, 3) angebracht ist.

3. Nagel nach Anspruch 2, bei dem der Kegel (9a) konisch nach außen gerichtet ist und 6 Zentimeter vom proximalen Ende angebracht ist.

4. Nagel nach Anspruch 1, bei dem der verlängerte Körper eine röhrenförmige Form hat.

## Revendications

1. Une broche intramédullaire (1) pour l'élongation du fémur adaptée pour l'insertion dans la cavité médullaire d'os entre le trochanter mineur et l'articulation du genou du fémur comprenant un corps linéaire élongé ayant à son extrémité proximale (3) et à son extrémité distale (2) des ouvertures de passage correspondantes (4, 5; 7,8) pour la réception de broches (P) ou de vis pour le fixage des extrémités (2, 3) au fémur **caractérisée en ce que** les ouvertures de passage proximales (4, 5) ont des axes longitudinaux s'étendant dans un premier plan et les ouvertures de passage distales (7, 8) ont des axes longitudinaux s'étendant dans un deuxième plan, lesdits premier et deuxième plans étant sensiblement perpendiculaires l'un à l'autre.

2. Une broche (1) selon la revendication 1 comprenant aussi un pivot conique (9a) logé au centre du corps élongé entre les extrémités proximale et distale (2, 3).

3. Une broche selon la revendication 2, dans laquelle le pivot (9a) est conique vers l'extérieur et logé à 6 centimètres de l'extrémité proximale.

4. Une broche selon la revendication 1, dans laquelle ledit corps élongé a une forme tubulaire.
